# NEW EUROPEAN PATENT SPECIFICATION

(11) **EP 2 235 652 B2**
(45) Date of publication and mention of the opposition decision: **15.06.2022**
(45) Mention of the grant of the patent: 17.10.2018
(21) Application number: 08859292.8
(22) Date of filing: 05.12.2008
(51) Int. Cl.: G16H 30/20

(54) **NAVIGATION IN A SERIES OF IMAGES**
NAVIGATION IN EINER BILDSERIE
NAVIGATION DANS UNE SÉRIE D'IMAGES

(30) Priority: 13.12.2007 EP 07123161
(43) Date of publication of application: 06.10.2010
(73) Proprietor: Koninklijke Philips N.V., 5656 AG Eindhoven (NL)
(72) Inventor: LOBREGT, Steven, NL-5656 AE Eindhoven (NL); PETERS, Joost, F., NL-5656 AE Eindhoven (NL); TJHANG, Alan, P., S., NL-5656 AE Eindhoven (NL)
(74) Representative: Philips Intellectual Property & Standards
(86) International application number: PCT/IB2008/055111
(87) International publication number: WO 2009/074931

(56) References cited:
- US-A1- 2004 161 139
- US-A1- 2004 249 291
- US-A1- 2005 111 761
- US-A1- 2006 159 325
- US-A1- 2007 061 726
- US-B2- 7 072 501
- GIUSEPPE SASSO ET AL: "A Visual Query-by-Example Image Database for Chest CT Images: Potential Role as a Decision and Educational Support Tool for Radiologists" JOURNAL OF DIGITAL IMAGING ; THE JOURNAL OF THE SOCIETY FOR COMPUTER APPLICATIONS IN RADIOLOGY, SPRINGER-VERLAG, NE, vol. 18, no. 1, 1 March 2005 (2005-03-01), pages 78-84, XP019368430 ISSN: 1618-727X
- LONG L R ET AL: "Image informatics at a national research center" COMPUTERIZED MEDICAL IMAGING AND GRAPHICS, PERGAMON PRESS, NEW YORK, NY, US, vol. 29, no. 2-3, 1 March 2005 (2005-03-01), pages 171-193, XP004774191 ISSN: 0895-6111

## Description

### FIELD OF THE INVENTION

The present invention relates to navigating in a series of related images, in particular in a series of medical images.

### BACKGROUND OF THE INVENTION

A common way of inspecting a large dataset comprising images is to load the dataset from a storage means and display the images one after the other. The images of interest within the dataset are found by scrolling through the large number of images. This is a time-consuming and cumbersome task, which includes navigating to the image(s) of interest in order to view an area of interest. When large datasets are being inspected, there is little help or assistance available to make it easier or faster to find the image(s) of interest.

The patent US 7 212 661 B2 describes a technique for generating navigational scout images, based upon a series of related images. One or more of the related images may be accessed by review of the navigational image. The navigational images are sample images selected from the related images from a large image dataset. The technique aims at facilitating identification of specific images in the image series,

### SUMMARY OF THE INVENTION

It is an object of the present invention to improve navigating in a series of related images.

In particular, it may be seen as an object of the present invention to provide a method, system and computer program product for fast and easy navigation in a series of related images.

In a first aspect, the invention provides a computer-implemented method of navigating in a series of related images as defined in claim 1.

The inventive method provides navigational entities created by segmentation of objects and by identification of the segmented objects; the navigational entities are used for user-friendly and fast navigation within the series of images. The segmented and identified objects being organs provide an intuitive or direct means of navigating to images of interest to a user.

The navigational entities may be stored together with, i.e. in the same storage means, and optionally interleaved with the series of related images. The related images may be images of a subject forming a series of images so that an object depicted in one or more of the related images may be an object depicted in one or more successive images in the series of images. The term "related images" is meant to denote images related to each other, such as different images of one subject. When the images are sequential in the meaning that they are ordered, e.g. as images taken along an axis of a subject, the term "successive images" is meant to describe images ordered in space by acquisition to form the series of images.

It should be noted that the method may furthermore comprise a preceding step of acquiring the series of related images and/or segmenting the image data. The creation of the navigational entities may be performed simultaneously with the acquisition of the images, or it may be performed after the acquisition of the series of related images has been concluded.

The term segmentation refers to the process of partitioning a digital image into multiple regions. The goal of segmentation is to simplify and/or change the representation of an image into something that is more meaningful and easier to analyze. Image segmentation is typically used to locate objects and boundaries (lines, curves, etc.) in images in order to separate objects of interest from each other and from the background.

The result of image segmentation may be a set of regions that collectively cover the entire image, or a set of contours extracted from the image. Each of the pixels in a region is similar with respect to some characteristic or computed property, such as color, intensity, or texture. Adjacent regions are significantly different with respect to the same characteristic(s).

Within medical imaging, typical examples of the use of image segmentation are: locating tumors and other pathologies, measuring tissue volumes, computer-guided surgery, diagnosis, treatment planning, study of anatomical structures.

A range of methods of segmentation is known. Any appropriate method of segmentation allowing subsequent identification of objects may be used. Such an appropriate method of segmentation could e.g. be chosen from amongst the following common methods of segmentation:
- thresholding, where the image is partitioned based on the value of each image element (pixel or voxel) being lower or higher than a certain threshold value. Multiple thresholds may be applied with the purpose to partition the image in more than two subsets of image elements. The value of the image elements can be a processed value too, for instance not the original measured value but another value derived from that. In this method of segmentation, thresholding is a point operation, where for the thresholding operation, only the value of the image element itself determines whether the element falls into one or the other partition;
- segmentation based on texture, where each element of the image has several values assigned to it. Thus, each image element represents a position in a multi-dimensional space, where the number of dimensions matches the number of values per image element. These values can represent many different aspects of the image, like brightness, color, gradients, statistical measures, etc. Segmentation in this case is usually called classification and is performed by separating clouds of points in the multi-dimensional space, or grouping those points using a clustering method;
- edge or surface-based segmentation. This method of segmentation identifies positions in an image that represent the transitions between those regions in an image that are different in some way. The gradient of a feature assigned to image elements, e.g. of the intensity of pixels or voxels, is usually computed and high values of this gradient indicate the location of edges or surfaces that form the boundary between regions;
- region-based segmentation. This method of segmentation aims at identifying groups of image elements on the basis of similarity with respect to some feature(s) of the image elements. The identification of groups of image elements is often implemented as a region-growing process, where, starting from a location or small area, image elements adjacent to this area are added to it if they fulfill a certain criterion of similarity to the area. Thus, the area grows until no more adjacent elements fulfill the criterion.

The above mentioned methods of segmentation are only a number of examples of segmentation techniques, and it should be understood that any other appropriate method of segmentation may be used.

According to the invention, each navigational entity comprises a first navigational part to be displayed and a second navigational part, said second navigational part comprising a reference to an image within the series of related images. Hereby, means are provided for navigating quickly within the series of images of the area of interest comprising the object, in that a reference or association is created between a first navigational part which is to be displayed and a particular image within the series of images.

Navigation is facilitated by displaying the first navigational part of the navigational entity on a display, providing means for letting a user choose said first navigational part and displaying the image referenced by the second navigational part of the navigational entity, if the first navigational part of said navigational entity is chosen by the user. Thus, by choosing a first navigational part of a navigational entity, a user may be able to view an image in the series of related images corresponding to the first navigational part. Therefore, easy switching between or navigation in different images of interest is facilitated, in particular when a number of navigational entities, appropriate for the objects depicted or shown in the images, is chosen.

The first navigational part of said navigational entity comprises an image representative of the identified object, an icon and/or a keyword representative of the identified object. This provides a user friendly presentation of the object identification and thereby a user-friendly user interface.

According to an embodiment of the invention, the first navigational part of said navigational entity is an image from the series of related images, shown in reduced size and/or in reduced resolution. In this case, the second navigational part should comprise a reference to the corresponding image in full resolution and full size in the series of images, so that the image in full resolution and full size may be displayed, if the reduced image is chosen by the user. It should be noted that the term "a reduced image" is meant to denote an image from the series of related images shown in reduced size and/or resolution. The navigational entities in the form of reduced images may be shown on a display together with an image in full size and full resolution.

According to a further embodiment, the series of related images may be displayed sequentially, beginning with the image referenced by the second navigational part. Thus, by choosing a navigational entity or a first navigational part of a navigational entity, a user may be able to view an image in the series of related images corresponding to the navigational entity and he/she may continue viewing subsequent or preceding images in the series of related images. Therefore, easy switching between or navigation in different images of interest is facilitated. The term "images referenced by the second navigational part" is meant to denote an image within the series of images to which the second navigational part refers.

According to another embodiment of the invention, the series of images comprises medical images obtained by scanning a subject, and wherein each image of the series of related images represents data acquired in a slice through the subject. The medical images may e.g. be obtained by computed tomography (CT), magnetic resonance imaging (MRI), ultrasound (US) or positron emission tomography (PET). Typically, the subject is a human or animal body and the objects identified comprise organs of said human or animal body. In this case, the related images form a sequence of images depicting slices of the body. Typically, hundreds or even thousands of images of a human body will be created for reviewing by clinicians to identify possible features of interest.

According to a second aspect, the invention relates to an apparatus for navigating in a series of images as defined in claim 5. The apparatus has advantages and additional aspects similar to those described above in connection with the method.

According to a third aspect, the invention relates to a computer program product for performing the steps of the method of the invention, when said computer program product is executed by a computer.

These and other aspects of the invention will be apparent from the following description with reference to the described embodiments.

### BRIEF DESCRIPTION OF THE FIGURES

The method and apparatus according to the invention will now be described in more detail with reference to the accompanying figures. The figures show one way of implementing the present invention and are not to be construed as being limited to other possible embodiments falling within the scope of the attached claim set. Throughout the figures, like reference numerals denote like elements.
Fig. 1 is a flow chart of a method according to the invention;
Fig. 2 is a diagram of an apparatus according to the invention;
Figs. 3 and 4 show different layouts of display means of an apparatus according to the invention;
Figs. 5a-5c and 6a-6b show examples of display means displaying medical images.

### DETAILED DESCRIPTION OF EMBODIMENTS

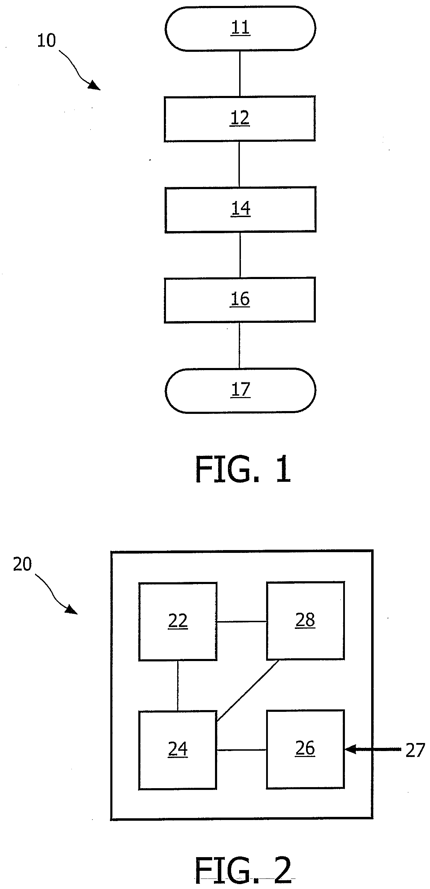

Figure 1 is a flow chart of a method 10 according to the invention. The method starts at step 11. The method 10 is to be performed after or simultaneously with the acquisition of a series of images. In the examples of the figures, the images are medical images. Such images may be obtained using e.g. computed tomography (CT), magnetic resonance imaging (MRI), ultrasound (US), and positron emission tomography (PET). Typically, a large number of images are acquired that may be reviewed by clinicians or radiologists to identify possible features of interest. In a medical context, these features may include anatomical regions, tissues, organs, anomalies that could be indicative of disease states.

The method 10 continues at step 12, wherein segmentation of objects depicted in images within one or more of the related images is performed. The segmentation may be any appropriate segmentation allowing subsequent identification of objects being organs.

Subsequently, the method 10 continues at step 14, wherein segmented objects are identified. Thus, step 14 may comprise analyzing the segmented objects and comparing them with predefined objects. Identification of an object may result in creation of a navigational entity comprising a first navigational part and a second navigational part. The first navigational part is to be displayed to a user, e.g. in a display of an apparatus, in a way that a user may choose the first navigational part by interacting with a user interface of the apparatus. The corresponding second navigational part of the navigational entity comprises a reference to an image within the series of images, e.g. a bookmark in the series of images, a pointer, etc. Hereby, quick access to the bookmarked or referenced images within the series of images is facilitated.

Typically, an object may be depicted in a range of successive images. In this case one of the images within the range of successive images is chosen as the image referenced by the second navigational part of the navigational entity; the navigational entity may render fast access to this bookmarked image, as explained below. The navigational entity may be an icon, a keyword or a piece of text or one of the images in the series of related images shown in reduced size and/or reduced resolution.

In the next step, step 16, navigation within the series of images is facilitated by means of the navigational entity/entities. As explained above, the creation of a navigational entity comprises the provision of a second navigational part, being a pointer, a bookmark or a reference to an appropriate image within the series of related images. It is well known to provide navigation in sets of data or images by means of such pointers, bookmarks or references.

The method 10 ends with step 17.

Figure 2 is a diagram of an apparatus 20 according to the invention for navigating in a series of images. The apparatus comprises a processor 24 functionally connected to a display 22 for displaying images, to a storage 26 and to a user interface 28. The apparatus moreover comprises an input 27 for receiving images functionally connected to the storage 26. The user interface 28 may also be functionally connected to the display 22. The apparatus may comprise other known means; however, for the sake of simplicity, only the features necessary in order to understand the invention are described.

Images may be loaded into the storage 26 as received via the input 27. The processor 24 may segment and identify objects present in one or more images in order to create navigational entities. Each navigational entity comprises a first navigational part to be displayed in the display 22 of the apparatus so as to allow a user to choose it. Each navigational entity also comprises a second navigational part comprising a pointer, a reference, an association or a bookmark to an image within the series of related images or a bookmark within the series of related images. In the case of medical images, such as images from a CT scan of a human body, the images correspond to slices through the body. The organs of the body, such as the liver, the spleen, the heart, the lungs, etc., may be depicted in a range of successive or otherwise related images. The processor 24 may segment objects depicted in the images and identify the segmented objects as e.g. the organs of the human body. Typically, an organ of the human body will appear in a number of successive or otherwise related images corresponding to a number of slices through the organ. The processor 24 may be arranged for choosing or selecting one image amongst the number of images as the reference or associated image or the bookmarked images. If an organ appears in e.g. N successive images, the processor 24 may choose the image in the middle, i.e. number N/2 or (N+1)/2 as the reference image or the bookmarked image. In this example, the reference image is an image in the middle of a set of N images. However, the reference image does not have to be in the middle of an object or close to the middle. Instead, the reference image could be at a position cutting through the object that is particularly representative of that object. Alternatively or additionally, the reference image could be the image through the center of gravity of the object, which image may be far from the middle depending on the shape of the object. Alternatively, the position of the reference image could be related to some specific shape feature of particular types of objects, e.g. a centerline or axis of symmetry, if a visually interesting location in a segmented object may be detected automatically or interactively, for instance as part of the segmentation process.

The first navigational parts may be shown in the display 22, alone or in conjunction with one or more images from the series of related images. Typically, the display 22 is divided into two or more display areas, wherein images from the series of related images may be shown in one display area whilst the first navigational parts may be shown in another display area. However, these display areas may be overlaid or one may be embedded in the other.

The apparatus 20 moreover comprises a user interface. This may comprise a keyboard or other navigational devices, such as a mouse associated with a display unit shown on the display. The user interface may enable a user to navigate in the series of images by highlighting or choosing, e.g. by clicks of the mouse, a navigational entity.

The apparatus 20 may be integrated in an apparatus for acquiring medical images, such as a CT-scanner, a PET-scanner, etc., in an apparatus for displaying images obtained by an acquisition apparatus or it may be implemented as a stand-alone apparatus. In the case where the apparatus is integrated in an apparatus for displaying images obtained by an acquisition apparatus, the display of the apparatus 20 may be the display of the apparatus for displaying images.

The terms "reference image", "associated image" and "bookmarked images" are meant to be synonymous and relate to an image within the series of related images to which a reference, pointer or bookmark refers.

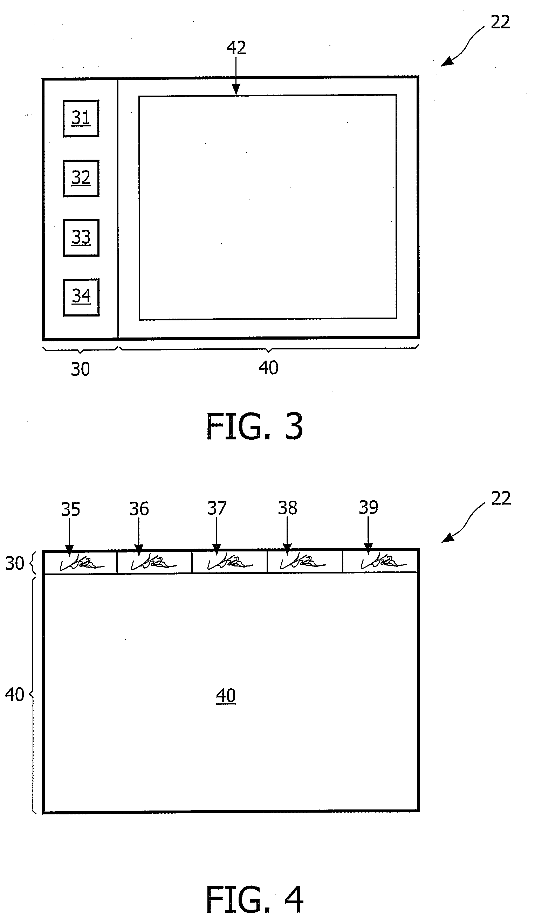

Figures 3 and 4 show different layouts of a display 22 of an apparatus according to the invention. The display 22 of figure 3 comprises a first display means 30 and a second display means 40. Typically, the first and second display means 30, 40 are different parts of the display 22, which are allocated to display different items. However, it is also conceivable that the first and second display means 30, 40 are separate display means. The first display means or the first display part 30 is arranged for displaying the first navigational parts of navigational entities. Such a first navigational part may be an image representative of the identified object, an icon, a keyword or a combination of these. The second display part 40 is arranged for showing an image 42 from the series of images.

When an object is depicted or shown in images from a range of successive images, one of the images in the range may be chosen as the image referred to by the second navigational part of the navigational entity. This referenced image in reduced size and/or reduced resolution may be the first navigational part of the navigational entity.

In figure 3, four of the first navigational parts 31-34 are shown. Of course any appropriate number of first navigational parts may be shown in the first display part 30. This number of first navigational parts may depend upon the size of the display part 30, the number of objects of interest in the series of images, the size of the first navigational parts, etc.

As described above, each navigational entity comprises a first and a second navigational part. The first navigational part is an image, text, icon or a combination thereof, corresponding to an image in the series of related images, whilst the second navigational part is a reference, a pointer or a bookmark pointing to said corresponding image in the series of related images. Thus, when a user chooses one of the first navigational parts 31-34 in the first display part 30, the apparatus 20 (see figure 2) uses the corresponding second navigational part to find the corresponding image in the storage 26 (see figure 2) for display thereof in the second display part 40 of the display 22. Thereby, user-friendly navigation within the series of related images is obtained.

Figure 4 shows an alternative layout of the display 22 of an apparatus according to the invention. In figure 4, the upper part of the display 22 constitutes the first display part 30. In this first display part 30 a number of keywords 35-39 are shown. The second display part 40 constitutes the remaining part of the display 22.

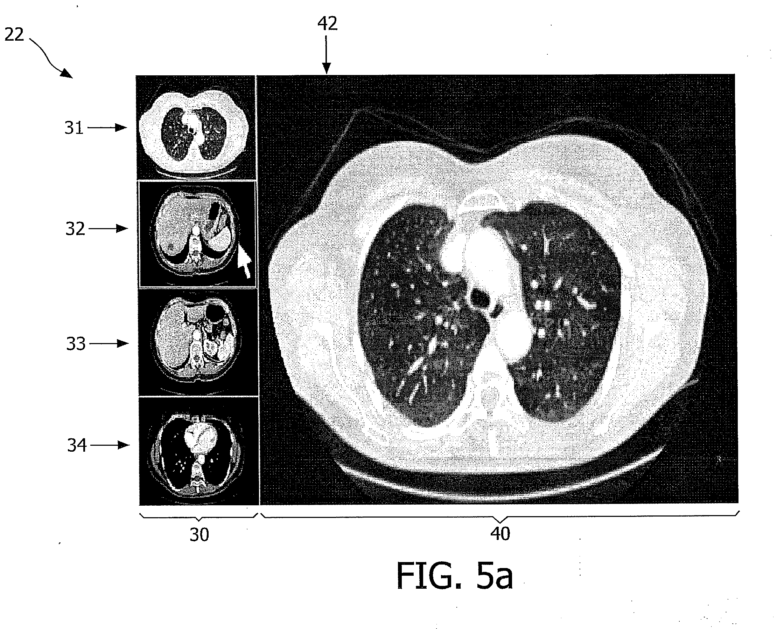

Figures 5a-5c show examples of display 22 displaying medical images. Figure 5a shows a layout of the display 22 corresponding to figure 3. In the first display means 30, four first navigational parts 31-34 are shown and in the second display means 40 an image 42 is shown. The image 42 corresponds to an upper first navigational part 31. Within a lower first navigational part 32 a white arrow is shown, and the boundary of the lower first navigational part 32 is highlighted. This is one example showing that a user has highlighted the lower first navigational part 32. If a user chooses the lower first navigational part 32, the corresponding image will be shown in the display part 40 instead of the image corresponding to the upper first navigational part 31.

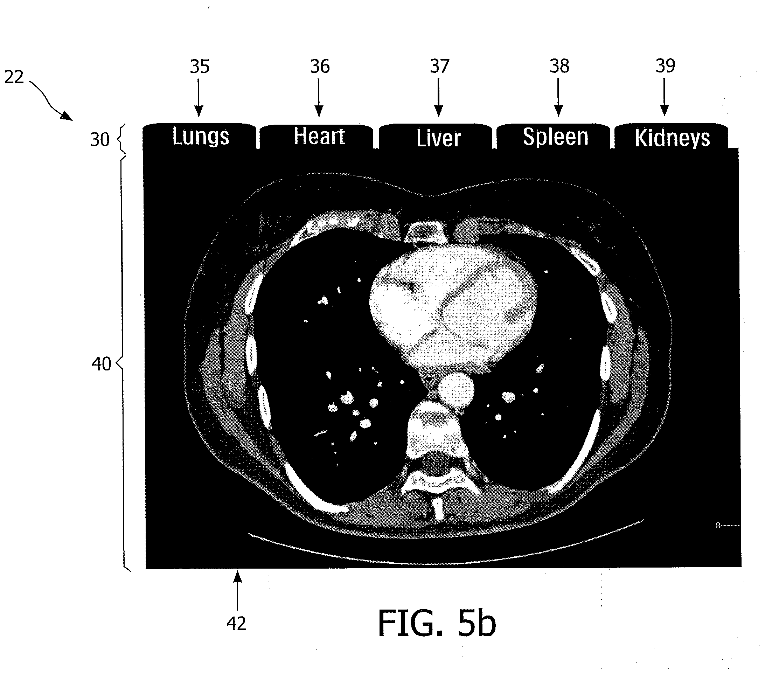

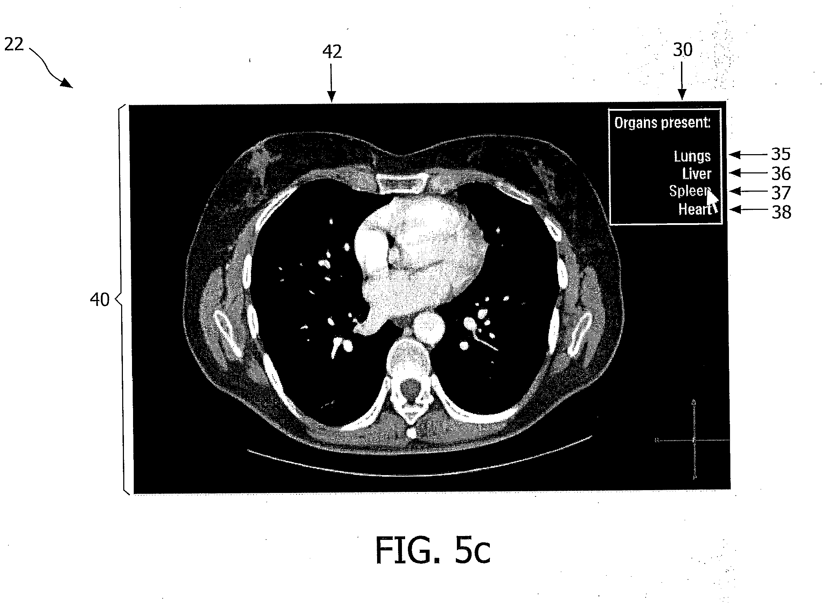

Figure 5b shows a layout of the display 22 corresponding to figure 4. In the first display means 30 five keywords 35-39 are shown and in the second display means 40 an image 42 is shown. If a user chooses one of the first navigational parts 35-39, the corresponding image will be shown in the display part 40. Figure 5c shows an alternative layout of the display means. In figure 5c, the first display means 30 is a relatively small square part in the upper right corner of the display 22. In this case, the first display means 30 is embedded within or integrated in the second display means 40.

The first display means 30 shows first navigational parts, viz. four keywords 35-38, whilst the second display means 40 shows an image 42. Again, if a user chooses one of the first navigational parts 35-38, an image corresponding to the chosen keyword 35-38 will be displayed in the second display means 40. A white arrow is shown in the first display means 30, indicating a part of the user interface arranged for letting a user choose one of the keywords 35-38 in order to activate the display in the second display means 40 of the image corresponding to the chosen keyword.

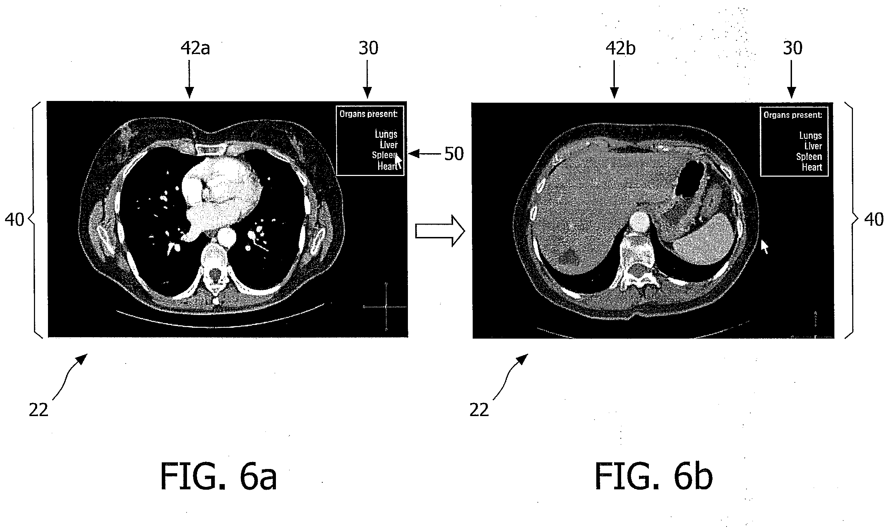

Figures 6a and 6b show two pictures of a display 22 of the layout shown in figure 5c. In figure 6a a picture 42a is displayed in the second display means 40. As in figure 5c, a first display means 30 comprising four keywords is also shown. A white arrow 50 is shown inside the first display means 30 indicating the user interface arranged for letting a user choose one of the keywords within the first display means 30. It can be seen that the keyword to which the arrow points is "Spleen". Figure 6b shows the display 22 after the keyword "Spleen" has been chosen by a user. In figure 6b, a picture 42b wherein the spleen may be seen, is shown in the second display means 40 of the display 22.

The invention can be implemented by means of hardware, software, firmware or any combination of these. The invention or some of the features thereof can also be implemented as software running on one or more data processors and/or digital signal processors or as software running in an apparatus for obtaining medical images or data, such as an apparatus for computed tomography (CT), magnetic resonance imaging (MRI), ultrasound (US) or positron emission tomography (PET).

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A computer-implemented method (10) of navigating in a series of related images, comprising the steps of:
- creating and storing navigational entities, each navigational entity relating to an object being an organ depicted in a range of successive images of the series of related images, wherein each navigational entity comprises a first navigational part to be displayed on a display (22) and a second navigational part, said second navigational part comprising a reference to an image within the series of related images, wherein the series of related images comprises medical images obtained by a scan of a subject and wherein each image of the series of related images represents data acquired in a slice through the subject;
- displaying the first navigational part of the navigational entity on the display (22);
- providing means for letting a user choose said first navigational part; and
- displaying the image referenced by the second navigational part of the navigational entity, when the first navigational part of said navigational entity is chosen by the user;
**characterized in that** the method further comprises, for each navigational entity:
- segmenting the object depicted in the range of successive images;
- identifying the segmented object;
- creating the first navigational part of the navigational entity to be representative of the identified object, wherein the first navigational part of said navigational entity comprises an image (31, 32, 33, 34), an icon and/or a keyword (35, 36, 37, 38, 39) representative of the identified object; and
- creating the second navigational part to comprise a reference to an image within the series of related images which comprises the identified object, wherein one of the images within the range of successive images is chosen as the image referenced by the second navigational part.

2. A method according to claim 1, wherein said first navigational part of said navigational entity is an image from the series of related images shown in reduced size and/or in reduced resolution.

3. A method according to any of the claims 1 or 2, wherein the images in the series of related images are displayed sequentially beginning with the image referenced by the second navigational part.

4. An apparatus (20) for navigating in a series of related images, comprising:
- processor means (24) for creating navigational entities, each navigational entity relating to an object being an organ depicted in a range of successive images of the series of related images, wherein each navigational entity comprises a first navigational part to be displayed on a display (22) and a second navigational part, said second navigational part comprising a reference to an image within the series of related images, wherein the series of related images comprises medical images obtained by a scan of a subject and wherein each image of the series of related images represents data acquired in a slice through the subject;
- storage means (26) for storing navigational entities created by the processor means,
- first display means (30) for displaying said first navigational part of said navigational entity;
- user interface means for letting a user choose said first navigational part; and
- second display means (40) for displaying the image referenced by the second navigational part of said navigational entity, when the first navigational part of said navigational entity is chosen by the user;
**characterized in that** said processor means (24) are arranged for, for each navigational entity:
- segmenting the object depicted in the range of successive images;
- identifying the segmented object;
- creating the first navigational part of the navigational entity to be representative of the identified object, wherein the first navigational part of said navigational entity comprises an image (31, 32, 33, 34), an icon and/or a keyword (35, 36, 37, 38, 39) representative of the identified object; and
- creating the second navigational part to comprise a reference to an image within the series of related images which comprises the identified object, wherein one of the images within the range of successive images is chosen as the image referenced by the second navigational part.

5. A computer program product for performing the steps of a method according to any of claims 1 to 3, when said computer program product is executed by a computer.

## Patentansprüche

1. Eine durch Computer verwirklichte Methode (10) der Navigation in einer Serie von miteinander verwandten Bildern, die folgenden Schritte umfassend:
- Erstellen und Speichern von Navigationseinheiten, wobei jede Navigationseinheit, die sich auf ein Objekt bezieht, ein Organ ist, das in einer Reihe aufeinanderfolgender Bilder aus der Reihe verwandter Bilder abgebildet ist, wobei jede Navigationseinheit einen ersten Navigationsteil umfasst, der auf einer Anzeige (22) angezeigt wird, und einen zweiten Navigationsteil, wobei der zweite Navigationsteil einen Verweis auf ein Bild innerhalb der Reihe verwandter Bilder umfasst, wobei die Reihe verwandter Bilder medizinische Bilder umfasst, die durch einen Scan eines Gegenstands erhalten wurden, und wobei jedes Bild der Reihe verwandter Bilder Daten darstellt, die in einem Schnitt durch den Gegenstand erhalten wurden;
- Abbildung des ersten Navigationsteils der Navigationseinheit im Display (22);
- Möglichkeit für den Nutzer, den genannten ersten Navigationsteil auszuwählen; und
- Abbildung des Bildes, welches vom zweiten Navigationsteil der Navigationseinheit in Bezug genommen wurde, sobald der erste Navigationsteil der genannten Navigationseinheit vom Nutzer ausgewählt wird;
**dadurch gekennzeichnet, dass** die Methode für jede Navigationseinheit weiterhin umfasst:
- Segmentierung des dargestellten Objekts in einer Reihe aufeinander folgender Bilder;
- Identifizierung des segmentierten Objekts;
- Erstellung des ersten Navigationsteils der Navigationseinheit, die das identifizierte Objekt darstellen soll, wobei der erste Navigationsteil der genannten Navigationseinheit ein Bild (31, 32, 33, 34), ein Symbol und/oder ein Schlüsselwort (35, 36, 37, 38, 39) umfasst, welches das identifizierte Objekt darstellt; und
- Erzeugen des zweiten Navigationsteils, um einen Verweis auf ein Bild innerhalb der Reihe verwandter Bilder zu umfassen, welches das identifizierte Objekt umfasst, wobei eines der Bilder innerhalb der Reihe aufeinanderfolgender Bilder als das Bild ausgewählt wird, auf das durch den zweiten Navigationsteil verwiesen wird.

2. Eine Methode nach Anspruch 1, wobei der erste Navigationsteil der genannten Navigationseinheit ein Bild aus der Serie von verwandten Bildern ist, das in geringerer Größe oder Auflösung gezeigt wird.

3. Eine Methode nach Anspruch 1 oder 2, wobei die Bilder in der Serie der verwandten Bilder in der Reihenfolge gezeigt werden, angefangen mit den Bildern, die vom zweiten Navigationsteil in Bezug genommen wurden.

4. Eine Vorrichtung (20) zum Navigieren in einer Serie von verwandten Bildern, umfassend:
- Verarbeitungsmittel (24) zum Erstellen von Navigationseinheiten, wobei jede Navigationseinheit, die sich auf ein Objekt bezieht, ein Organ ist, das in einer Reihe aufeinanderfolgender Bilder der Reihe verwandter Bilder abgebildet ist, wobei jede Navigationseinheit einen ersten Navigationsteil umfasst, der auf einer Anzeige (22) angezeigt wird, und einen zweiten Navigationsteil, wobei der zweite Navigationsteil einen Verweis auf ein Bild innerhalb der Reihe verwandter Bilder umfasst, wobei die Reihe verwandter Bilder medizinische Bilder umfasst, die durch einen Scan eines Gegenstands erhalten wurden, und wobei jedes Bild der Reihe verwandter Bilder Daten darstellt, die in einem Schnitt durch den Gegenstand erhalten wurden;
- Speichermittel (26) zum Speichern von Navigationseinheiten, die durch das Verarbeitungsmittel erstellt wurden,
- ein erstes Darstellungsmittel (30) zum Darstellen des genannten Navigationsteils der genannten Navigationseinheit;
- Schnittstelle für den Nutzer, um den genannten ersten Navigationsteil auszuwählen; und
- ein zweites Abbildungsmittel (40), welches vom zweiten Navigationsteil der Navigationseinheit in Bezug genommen wurde, sobald der erste Navigationsteil der genannten Navigationseinheit vom Nutzer ausgewählt wird;
**dadurch gekennzeichnet, dass** das genannte Verarbeitungsmittel (24) für jede Navigationseinheit angeordnet ist:
- Segmentierung des dargestellten Objekts in einer Reihe aufeinander folgender Bilder;
- Identifizierung des segmentierten Objekts;
- Erstellung des ersten Navigationsteils der Navigationseinheit, die das identifizierte Objekt darstellen soll, wobei der erste Navigationsteil der genannten Navigationseinheit ein Bild (31, 32, 33, 34), ein Symbol und/oder ein Schlüsselwort (35, 36, 37, 38, 39) umfasst, welches das identifizierte Objekt darstellt; und
- Erzeugen des zweiten Navigationsteils, um einen Verweis auf ein Bild innerhalb der Reihe verwandter Bilder zu umfassen, welches das identifizierte Objekt umfasst, wobei eines der Bilder innerhalb der Reihe aufeinanderfolgender Bilder als das Bild ausgewählt wird, auf das durch den zweiten Navigationsteil verwiesen wird.

5. Ein Produkt eines Computerprogramms, um die Schritte einer Methode nach einem der Ansprüche 1 bis 3 auszuführen, wenn das genannte Produkt eines Computerprogramms von einem Computer ausgeführt wird.

## Revendications

1. Procédé mis en œuvre par ordinateur (10) de navigation dans une série d'images liées, comprenant les étapes consistant à:
- créer et stocker des entités de navigation, chaque entité de navigation se rapportant à un objet étant un organe représenté dans une gamme d'images successives de la série d'images associées, dans lequel chaque entité de navigation comprend une première partie de navigation à afficher sur un écran (22) et une seconde partie de navigation, ladite seconde partie de navigation comprenant une référence à une image dans la série d'images associées, dans lequel la série d'images associées comprend des images médicales obtenues par un balayage d'un sujet et dans lequel chaque image de la série d'images associées représente des données acquises dans une tranche à travers le sujet;
- afficher la première partie de navigation de l'entité de navigation sur l'affichage (22);
- fournir des moyens pour permettre à un utilisateur de choisir ladite première partie de navigation; et
- afficher l'image référencée par la deuxième partie de navigation de l'entité de navigation, lorsque la première partie de navigation de ladite entité de navigation est choisie par l'utilisateur;
**caractérisé en ce que** le procédé comprend en outre, pour chaque entité de navigation:
- segmenter l'objet représenté dans la série d'images successives;
- identifier l'objet segmenté;
- créer la première partie de navigation de l'entité de navigation pour qu'elle soit représentative de l'objet identifié, dans laquelle la première partie de navigation de ladite entité de navigation comprend une image (31, 32, 33, 34), une icône et/ou un mot clé (35, 36, 37, 38, 39) représentatif de l'objet identifié; et
- créer la deuxième partie de navigation pour comprendre une référence à une image dans la série d'images liées qui comprend l'objet identifié, dans lequel l'une des images dans la gamme d'images successives est choisie comme l'image référencée par la deuxième partie de navigation.

2. Procédé selon la revendication 1, dans lequel ladite première partie de navigation de ladite entité de navigation est une image de la série d'images associées montrées en taille réduite et/ou en résolution réduite.

3. Procédé selon l'une quelconque des revendications 1 ou 2, dans lequel les images de la série d'images liées sont affichées séquentiellement en commençant par l'image référencée par la deuxième partie de navigation.

4. Un appareil (20) pour naviguer dans une série d'images liées, comprenant:
- un moyen processeur (24) pour créer et stocker des entités de navigation, chaque entité de navigation se rapportant à un objet étant un organe représenté dans une gamme d'images successives de la série d'images associées, dans lequel chaque entité de navigation comprend une première partie de navigation à afficher sur un écran (22) et une seconde partie de navigation, ladite seconde partie de navigation comprenant une référence à une image dans la série d'images associées, dans lequel la série d'images associées comprend des images médicales obtenues par un balayage d'un sujet et dans lequel chaque image de la série d'images associées représente des données acquises dans une tranche à travers le sujet;
- des moyens de stockage (26) pour stocker des entités de navigation créées par les moyens de traitement,
- un premier moyen d'affichage (30) pour afficher ladite première partie de navigation de ladite entité de navigation;
- des moyens d'interface utilisateur pour permettre à un utilisateur de choisir ladite première partie de navigation; et
- des seconds moyens d'affichage (40) pour afficher l'image référencée par la deuxième partie de navigation de ladite entité de navigation, lorsque la première partie de navigation de ladite entité de navigation est choisie par l'utilisateur;
**caractérisé en ce que** lesdits moyens de traitement (24) sont disposés pour, pour chaque entité de navigation:
- segmenter l'objet représenté dans la série d'images successives;
- identifier l'objet segmenté;
- créer la première partie de navigation de l'entité de navigation pour qu'elle soit représentative de l'objet identifié, dans laquelle la première partie de navigation de ladite entité de navigation comprend une image (31, 32, 33, 34), une icône et/ou un mot clé (35, 36, 37, 38, 39) représentatif de l'objet identifié; et
- créer la deuxième partie de navigation pour comprendre une référence à une image dans la série d'images liées qui comprend l'objet identifié, dans lequel l'une des images dans la gamme d'images successives est choisie comme l'image référencée par la deuxième partie de navigation.

5. Produit de programme informatique pour réaliser les étapes d'un procédé selon l'une quelconque des revendications 1 à 3, lorsque ledit produit de programme informatique est exécuté par un ordinateur.
